# EUROPEAN PATENT SPECIFICATION

(11) **EP 1 897 491 B1**
(45) Date of publication and mention of the grant of the patent: **29.12.2010**
(21) Application number: 07012830.1
(22) Date of filing: 29.06.2007
(51) Int. Cl.: A61B 5/053, A61B 5/00, G06F 19/00

(54) **Body compositions indicator estimation system**
Schätzsystem für einen Indikator für Körperzusammensetzungen
Système d'estimation indicateur de compositions corporelles

(30) Priority: 06.09.2006 JP 2006242089
(43) Date of publication of application: 12.03.2008
(73) Proprietor: Tanita Corporation, Tokyo 174-8630 (JP)
(72) Inventor: Sato, Tomio, Tokyo 174-8630 (JP); Hasegawa, Hiroki, Tokyo 174-8630 (JP); Ikeda, Masanori, Tokyo 174-8630 (JP)
(74) Representative: Müller-Boré & Partner Patentanwälte

(56) References cited:
- EP-A- 1 055 396
- WO-A-01/76220
- WO-A-01/97154

## Description

### BACKGROUND OF THE INVENTION

### (i) Field of the Invention

The present invention relates to a body composition indicator estimation system which estimates body composition indicators (which are indicators about components and tissues constituting a body, such as a body fat percentage, visceral fat area, muscle amount, body water amount and bone mass).

### (ii) Description of the Related Art

Conventional body composition indicator estimation devices acquire body indicators such as a body weight, body height, age, sex and a body impedance through input or measurement and estimate a body composition indicator based on the acquired body indicators.

As an example of such body composition indicator estimation devices, a body composition measuring device is disclosed in Patent Literature 1. This body composition measuring device inputs a body height by an input unit, measures a body weight by a body weight measuring unit, acquires a bioelectrical impedance (body impedance) through measurement by current applying means, voltage measuring means and bioelectrical impedance calculating means, and calculates a body density first based on these acquired body height, body weight and bioelectrical impedance, estimates a body fat percentage (body composition indicator) through calculation based on the calculated body density and then estimates a body fat mass (body composition indicator) or a fat free mass (body composition indicator) through calculation based on the calculated body fat percentage and the acquired body weight by an arithmetic control unit.

### Patent Literature 1

Japanese Patent Application Laid-Open No. 329412/2004

Meanwhile, manufacturers of body composition indicator estimation devices develop estimation equations with higher accuracy of estimation of body composition indicator one after another and provide (sell) body composition indicator estimation devices which estimate a body composition indicator by calculation using an estimation equation with higher accuracy one after another. On the other hand, users who demand higher accuracy of estimation of body composition indicator must purchase body composition indicator estimation devices provided by manufacturers one after another and register input data (body indicators which are acquired not through measurements and used to calculate a body composition indicator) again every time they purchase the body composition indicator estimation devices, thereby imposing a burden on the users financially and in terms of ease of use of the body composition indicator estimation device.

WO 2004/069164 A (D1) discloses a system for predicting blood glucose values in a patient which includes a remote wireless non-invasive spectral device, the remote wireless non-invasive spectral device generating a spectral scan of a body part of the patient. Also included are a remote invasive device and a central processing device. The remote invasive device generates a constituent value for the patient, while the central processing device predicts a blood glucose value for the patient based upon the spectral scan and the constituent value.
Thus, the present invention has been conceived to solve the above problem of the prior art. An object of the present invention is to provide a body composition indicator estimation system which imposes a little burden on users in using the system.
This object is solved by a body composition indicator estimation system having the features of claim 1. Preferred embodiments of the invention are defined in the dependent claims.

### SUMMARY OF THE INVENTION

A body composition indicator estimation system of the present invention comprises:
a body indicator acquiring unit which acquires a body indicator,
a body indicator management unit which manages the body indicator acquired by the body indicator acquiring unit, and
a body indicator confirmation terminal unit which outputs the body indicator managed by the body indicator management unit to a user, these units being connected via a telecommunication network, wherein
the body indicator acquiring unit comprises:
basic body indicator measuring means for measuring basic body indicators which provide a correlation with a body composition indicator, and
basic body indicator relay means for transmitting the basic body indicators measured by the basic body indicator measuring means to the body indicator management unit via the telecommunication network,
the body indicator management unit comprises:
body composition indicator acquiring means for calculating a body composition indicator based on the basic body indicators transmitted from the basic body indicator relay means in accordance with a correlation equation of the basic body indicators and the body composition indicator which is stored in the body composition indicator acquiring means and transmitting the calculated body composition indicator to the body indicator confirmation terminal unit via the telecommunication network, and
correlation equation changing means for changing the correlation equation stored in the body composition indicator acquiring means, and
the body indicator confirmation terminal unit comprises:
body composition indicator confirmation terminal means for outputting the body composition indicator transmitted from the body composition indicator acquiring means to the user.

Further, the basic body indicator relay means comprises a mobile relay key which stores the basic body indicators measured by the basic body indicator measuring means and a user ID which corresponds to the basic body indicators and a relay terminal which transmits the basic body indicators and user ID stored in the mobile relay key to the body composition indicator acquiring means via the telecommunication network, the basic body indicator measuring means measures the basic body indicators by working with the mobile relay key, and the mobile relay key is portable and formed independently of the basic body indicator measuring means and the relay terminal.

Further, the basic body indicator measuring means further comprises a mobile ID transmission key which transmits a user ID, the basic body indicator measuring means measures the basic body indicators by working with the mobile ID transmission key, the basic body indicator relay means comprises a relay terminal which stores the basic body indicators measured by the basic body indicator measuring means and a user ID which corresponds to the basic body indicators and transmits the stored basic body indicators and user ID to the body composition indicator acquiring means via the telecommunication network, and the mobile ID transmission key is portable and formed independently of the basic body indicator measuring means and the relay terminal.

Further, the body composition indicator confirmation terminal means requests a body composition indicator corresponding to the user ID from the body composition indicator acquiring means via the telecommunication network, and the body composition indicator acquiring means comprises a basic body indicator storage server which stores other basic body indicators which provide a correlation with a body composition indicator together with the basic body indicators measured by the basic body indicator measuring means and a user ID which corresponds to the basic body indicators, a body composition indicator computation server which calculates a body composition indicator based on the basic body indicators transmitted from the relay terminal together with the user ID via the telecommunication network and the basic body indicators stored in the basic body indicator storage server together with the user ID in accordance with the correlation equation, and a body composition indicator storage server which stores the body composition indicator calculated by the body composition indicator computation server and the user ID corresponding to the body composition indicator and transmits the stored body composition indicator based on a request from the body composition indicator confirmation terminal means.

The body composition indicator estimation system of the present invention measures basic body indicators which provide a correlation with a body composition indicator by the basic body indicator measuring means, transmits the measured basic body indicators to the body indicator management unit via the telecommunication network by the basic body indicator relay means, calculates a body composition indicator based on the transmitted basic body indicators in accordance with a correlation equation of the basic body indicators and body composition indicator which can be changed on the administrator side and transmits the calculated body composition indicator to the body indicator confirmation terminal unit via the telecommunication network by the body composition indicator acquiring means, and outputs the transmitted body composition indicator to a user by the body composition indicator confirmation terminal means. Thus, the present system can always provide the best body composition indicator without imposing a burden, such as purchase of another system or re-registration of the same input data, on a user.

Further, in the body composition indicator estimation system of the present invention, the basic body indicator measurement device measures the basic body indicators by working with the mobile relay key or mobile ID transmission key, the correlation equation for calculating the body composition indicator can be changed on the administrator side, and data is processed based on a user ID from the mobile relay key or mobile ID transmission key. Thus, the present system can always provide the best body composition indicator more conveniently without imposing a burden on a user in using the system.

### BRIEF DESCRIPTION OF THE DRAWINGS

Fig. 1 is a system configuration diagram which illustrates the configuration of a body composition indicator estimation system of the present invention functionally.
Fig. 2 is a system configuration diagram which illustrates the configuration of a body composition indicator estimation system of the present invention (Example 1).
Fig. 3 is a flowchart illustrating the first half of a flow of data based on the operation of the body composition indicator estimation system of the present invention (Example 1).
Fig. 4 is a flowchart illustrating the second half of a flow of data based on the operation of the body composition indicator estimation system of the present invention (Example 1).
Fig. 5 is a system configuration diagram which illustrates the configuration of a body composition indicator estimation system of the present invention (Example 2).
Fig. 6 is a flowchart illustrating the first half of a flow of data based on the operation of the body composition indicator estimation system of the present invention (Example 2).
Fig. 7 is a flowchart illustrating the second half of a flow of data based on the operation of the body composition indicator estimation system of the present invention (Example 2).

### DETAILED DESCRIPTION OF THE PREFERRED EMBODIMENTS

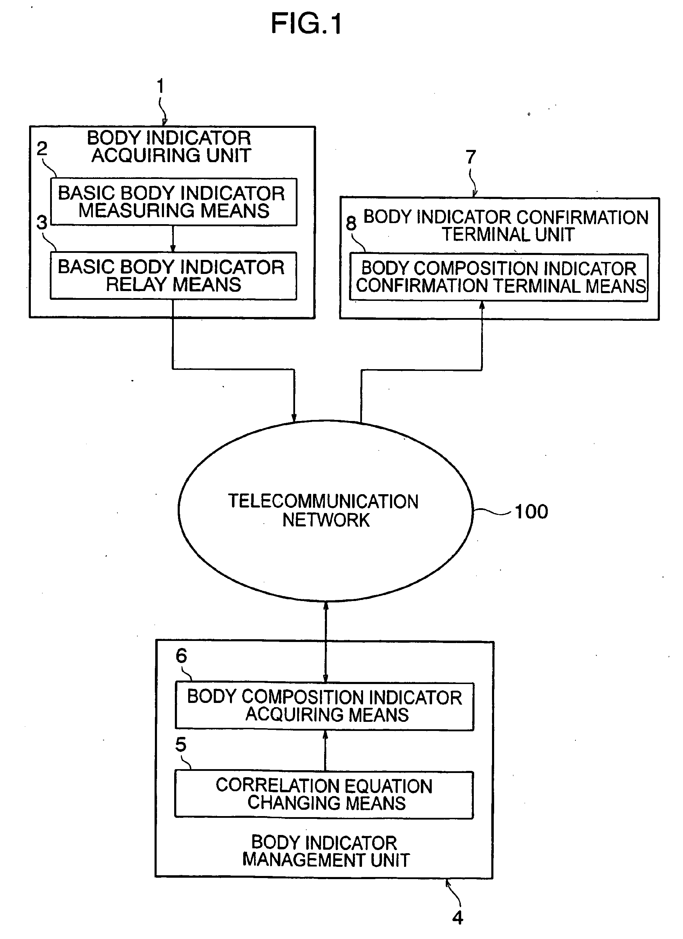

As shown in a system configuration diagram in Fig. 1 illustrating the configuration of a body composition indicator estimation system functionally, a body composition indicator estimation system of the present invention comprises a body indicator acquiring unit 1 which comprises basic body indicator measuring means 2 and basic body indicator relay means 3 and acquires a body indicator, a body indicator management unit 4 which comprises correlation equation changing means 5 and body composition indicator acquiring means 6 and manages the body indicator acquired by the body indicator acquiring unit 1, and a body indicator confirmation terminal unit 7 which comprises body composition indicator confirmation terminal means 8 and outputs the body indicator managed by the body indicator management unit 4 to a user. These units 1, 4 and 7 are connected via a telecommunication network (such as the Internet or a cellular phone communication network) 100. The body indicator acquiring unit 1 and the body indicator confirmation terminal unit 7 are used on the user side which is provided with estimation of body composition indicator, and the body indicator management unit 4 is used on the administrator (such as a provider) side which provides estimation of body composition indicator.

The basic body indicator measuring means 2 measures basic body indicators (such as a body impedance and a body weight) which provide a correlation with a body composition indicator.

The basic body indicator relay means 3 transmits the basic body indicators measured by the basic body indicator measuring means 2 to the body indicator management unit 4 via the telecommunication network 100.

The body composition indicator acquiring means 6 stores a correlation equation of basic body indicators and body composition indicator, calculates a body composition indicator by substituting the basic body indicators transmitted from the basic body indicator relay means 3 into the stored correlation equation of basic body indicators and body composition indicator, and transmits the calculated body composition indicator to the body indicator confirmation terminal unit 7 via the telecommunication network 100. The body composition indicator acquiring means 6 may calculate the body composition indicator by storing other basic body indicators (such as a body height, age and sex) which provide a correlation with the body composition indicator together with the basic body indicators measured by the basic body indicator measuring means 2 in advance and substituting the stored basic body indicators and the basic body indicators transmitted from the basic body indicator relay means 3 into the correlation equation of basic body indicators and body composition indicator.

The correlation equation changing means 5 makes the correlation equation of basic body indicators and body composition indicator which is stored in the body composition indicator acquiring means 6 changeable on the administrator side.

The body composition indicator confirmation terminal means 8 outputs the body composition indicator transmitted from the body composition indicator acquiring means 6 to a user.

According to the thus constituted body composition indicator estimation system, basic body indicators which provide a correlation with a body composition indicator are measured by the basic body indicator measuring means 2, the measured basic body indicators are transmitted to the body indicator management unit 4 via the telecommunication network 100, a body composition indicator is calculated by substituting the transmitted basic body indicators (or the transmitted basic body indicators and basic body indicators stored in the body composition indicator acquiring means 6) into a correlation equation of basic body indicators and body composition indicator and the calculated body composition indicator is transmitted to the body indicator confirmation terminal unit 7 via the telecommunication network 100 by the body composition indicator acquiring means 6, the transmitted body composition indicator is output to a user by the body composition indicator confirmation terminal means 8, and a change of the stored correlation equation between basic body indicators and body composition indicator is made changeable on the administrator side by the correlation equation changing means 5. Accordingly, the present system can always provide the best body composition indicator without imposing a burden, such as purchase of another system or re-registration of the same input data, on a user.

Hereinafter, embodiments in the above form will be described specifically.

### Example 1

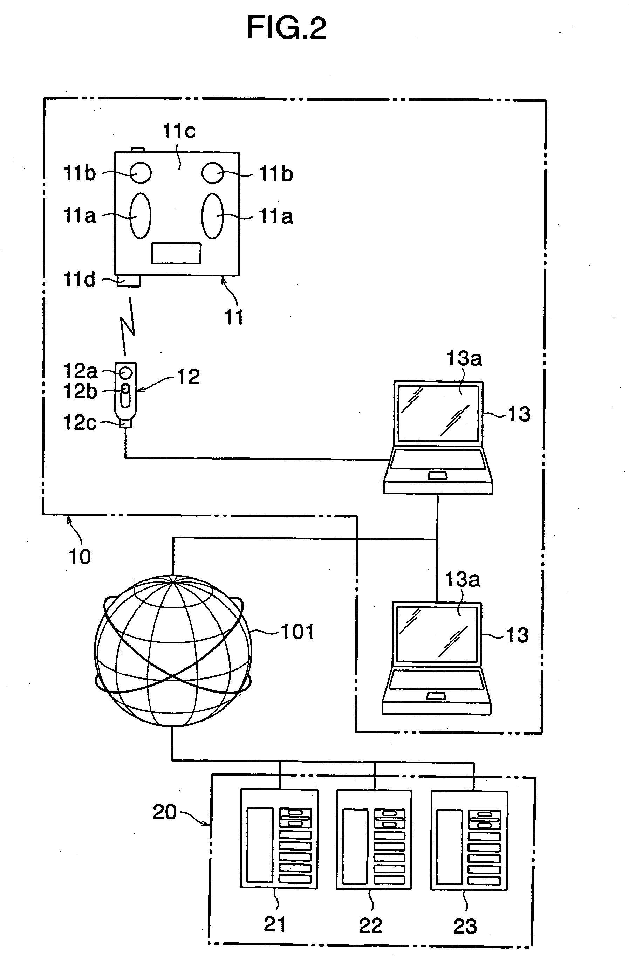

First, the configuration of a body composition indicator estimation system as Example 1 of the present invention will be specifically described by use of a system configuration diagram in Fig. 2 illustrating the configuration of the body composition indicator estimation system.

The body composition indicator estimation system in Example 1 comprises a basic body indicator measurement device 11, a mobile relay key 12 and a PC (Personal Computer) 13 on a user side 10 and comprises a basic body indicator storage server 21, a body composition indicator computation server 22 and a body composition indicator storage server 23 on an administrator (provider) side 20. Further, the PC 13 and the servers 21, 22 and 23 are connected to an Internet 101 via a router (not shown).

The basic body indicator measurement device 11 corresponds to the aforementioned basic body indicator measuring means 2 and has current-passing electrodes 11a and measuring electrodes 11b on the platform 11c of the scale and measures a body impedance and a body weight as basic body indicators. Further, the basic body indicator measurement device 11 also has wireless communication means 11d using an infrared ray, low-power radio or the like and communicates data (user ID, basic body indicators (body impedance, body weight)) with the mobile relay key 12 thereby.

The mobile relay key 12 corresponds to a part of the aforementioned basic body indicator relay means 3 and stores the basic body indicators (body impedance, body weight) measured by the basic body indicator measurement device 11 and a user ID for identifying a user corresponding to the basic body indicators (body impedance, body weight) and is so formed to be portable. More specifically, the mobile relay key 12 is approximately the size of a portable lighter, has wireless communication means using an infrared ray, low-power radio or the like and communicates the data (user ID, basic body indicators (body impedance, body weight)) with the basic body indicator measurement device 11 thereby. Further, the mobile relay key 12 has storage means for storing the communication-related data (user ID, basic body indicators (body impedance, body weight)). In addition, the mobile relay key 12 has an USB terminal 12c which can be slid out of or slid into the mobile relay key 12 by sliding a slide key 12b and is thereby connected to the PC 13 to transmit the stored data (user ID, basic body indicators (body impedance, body weight)).

The PC 13 corresponds to a part of the aforementioned basic body indicator relay means 3 and is a relay terminal which transmits the data (user ID, basic body indicators (body impedance, body weight)) stored in the mobile relay key 12 to the body composition indicator computation server 22 via the Internet 101. Further, the PC 13 corresponds to the aforementioned body composition indicator confirmation terminal means 8 and requests a body composition indicator (body fat percentage) corresponding to the user ID from the body composition indicator storage server 23 via the Internet 101 and displays the body composition indicator (body fat percentage) transmitted from the body composition indicator storage server 23 to a user. More specifically, the PC 13 communicates the data (user ID, basic body indicators (body impedance, body weight)) with the mobile relay key 12, communicates the data (user ID, basic body indicators (body impedance, body weight)) with the body composition indicator computation server 22 and communicates the data (user ID and body composition indicator (body fat percentage)) with the body composition indicator storage server 23 in accordance with a software recording medium for communicating the data (user ID, basic body indicators (body impedance, body weight)), and displays the acquired body composition indicator (body fat percentage) in a display section 13a.

The basic body indicator storage server 21 corresponds to a part of the aforementioned body composition indicator acquiring means 6 and stores other basic body indicators (body height, sex, age) which provide a correlation with the body composition indicator together with the basic body indicators (body impedance, body weight) measured by the basic body indicator measurement device 11 and a user ID for identifying a user corresponding to the basic body indicators (body height, sex, age) and communicates the data with the body composition indicator computation server 22. The basic body indicators (body height, sex, age) and user ID to be stored can be input by various techniques. For example, they can be input through the input section of the basic body indicator storage server 21 or input from the user side 10 via the Internet 101.

The body composition indicator computation server 22 corresponds to a part of the aforementioned body composition indicator acquiring means 6 and stores a correlation equation of basic body indicators (body impedance, body weight, body height, sex, age) and body composition indicator (body fat percentage), calculates a body composition indicator (body fat percentage) by substituting the basic body indicators (body impedance, body weight) transmitted from the PC 13 together with the user ID via the Internet 101 and the basic body indicators (body height, sex, age) transmitted from the basic body indicator storage server 21 together with the user ID into the correlation equation of basic body indicators (body impedance, body weight, body height, sex, age) and body composition indicator (body fat percentage), and transmits the calculated body composition indicator (body fat percentage) to the body composition indicator storage server 23 together with the user ID. Further, the body composition indicator computation server 22 corresponds to the aforementioned correlation equation changing means 5 and makes the stored correlation equation of basic body indicators (body impedance, body weight, body height, sex, age) and body composition indicator (body fat percentage) changeable on the administrator (provider) side 20. It can change constants in the correlation equation of basic body indicators (body impedance, body weight, body height, sex, age) and body composition indicator (body fat percentage) or replace the correlation equation with a correlation equation of basic body indicators (body impedance, body weight, body height, sex, age) and body composition indicator (visceral fat area).

The body composition indicator storage server 23 stores the body composition indicator (body fat percentage) and user ID for identifying a user corresponding to the body composition indicator (body fat percentage) which have been transmitted from the body composition indicator computation server 22 and transmits the stored body composition indicator (body fat percentage) based on a request from the PC 13.

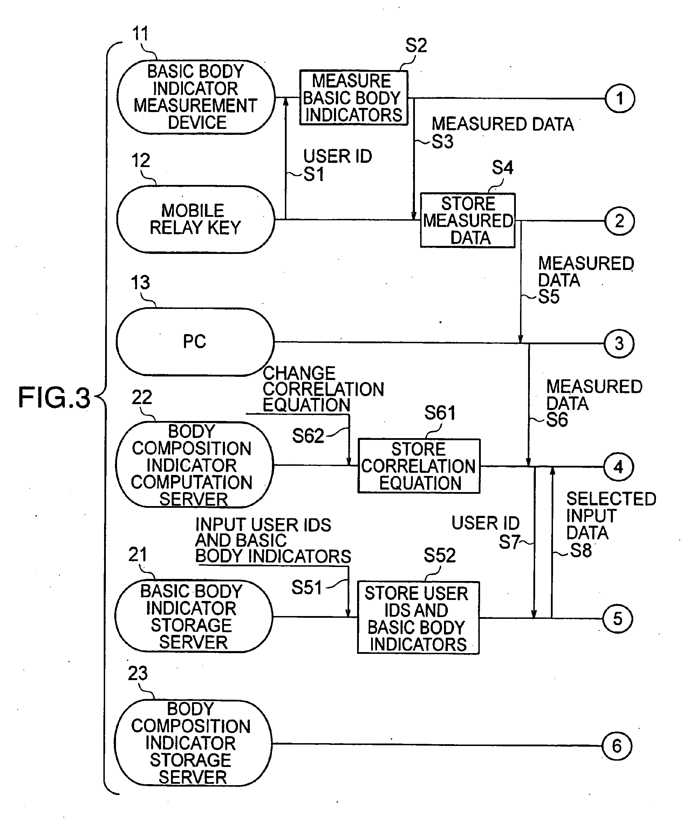

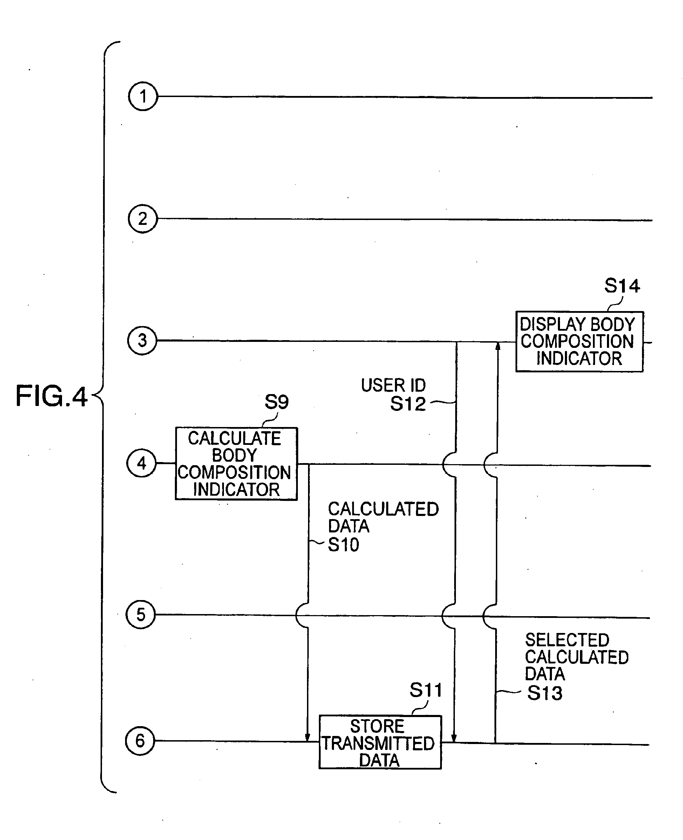

Next, a flow of data based on the operation of the body composition indicator estimation system according to the present invention will be specifically described by use of flowcharts in Figs. 3 and 4 illustrating a flow of data based on the operation of the body composition indicator estimation system.

Figs. 3 and 4 illustrate, from the top to the bottom, flows of processes in the basic body indicator measurement device 11, mobile relay key 12, PC 13, body composition indicator computation server 22, basic body indicator storage server 21 and body composition indicator storage server 23 and illustrate flows of data based on mutual communication by connecting these process routes by lines. Further, although an end terminal is not particularly illustrated, the following processes are repeated as appropriate.

First, when a user (subject) presses the communication button 12a of the mobile relay key 12, the mobile relay key 12 transmits data about a user ID and the like to the basic body indicator measurement device 11 wirelessly (STEP S1).

Then, the basic body indicator measurement device 11 is activated automatically based on the transmitted data, measures basic body indicators (body weight, body impedance) (STEP S2), and transmits data about the measured basic body indicators (body weight, body impedance) and a user ID corresponding to the basic body indicators (i.e. user ID transmitted from the mobile relay key 12) to the mobile relay key 12 wirelessly (STEP S3).

Then, the mobile relay key 12 receives and stores the transmitted data (STEP S4). Then, when the user (subject) slides the USB terminal 12c out of the mobile relay key 12 by sliding the slide key 12b and connects the terminal 12c to a USB port of the PC 13 which is ready to receive the data, the mobile relay key 12 transmits the stored data to the PC 13 (STEP S5).

Then, the PC 13 receives the transmitted data and transmits the data to the body composition indicator computation server 22 via the Internet 101 (STEP S6).

Then, when the body composition indicator computation server 22 receives the data from the PC 13 via the Internet 101, it transmits data about the user ID to the basic body indicator storage server 21 (STEP S7).

Then, when the basic body indicator storage server 21 receives the transmitted data about the user ID, it selects basic body indicators (body height, sex, age) corresponding to the transmitted user ID from input data which has been input (STEP S51) and stored (STEP S52) in advance, i.e. data about basic body indicators (body height, sex, age) and user IDs corresponding to the basic body indicators and transmits the selected input data, i.e. data about the basic body indicators (body height, sex, age) and the user ID corresponding to the basic body indicators to the body composition indicator computation server 22 (STEP S8).

Then, when the body composition indicator computation server 22 receives the transmitted data, it calculates a body composition indicator (body fat percentage) by substituting the basic body indicators (body impedance, body weight) transmitted from the PC 13 via the Internet 101 in STEP S6 and the basic body indicators (body height, sex, age) transmitted from the basic body indicator storage server 21 in STEP S8 into a correlation equation of basic body indicators (body impedance, body weight, body height, sex, age) and body composition indicator (body fat percentage) which has been stored (STEP S61) in advance (STEP S9) and transmits data about the calculated body composition indicator (body fat percentage) and the user ID corresponding to the body composition indicator to the body composition indicator storage server 23 (STEP S10). The correlation equation stored in STEP S61 is changed as appropriate by changing constants in the correlation equation of basic body indicators (body impedance, body weight, body height, sex, age) and body composition indicator (body fat percentage) or replacing the correlation equation with a correlation equation of basic body indicators (body impedance, body weight, body height, sex, age) and body composition indicator (visceral fat area) on the administrator (provider) side (STEP S62).

Then, the body composition indicator storage server 23 receives and stores the transmitted data (STEP S11).

Then, when the user ID of the target user (subject) is entered in the PC 13 by the user (subject or a person who supervises the subject (such as a doctor or a family member living separately)) to view the measurement results, the PC 13 transmits data about the entered user ID to the body composition indicator storage server 23 (STEP S12).

Then, when the body composition indicator storage server 23 receives the transmitted data, it selects a body composition indicator (body fat percentage) corresponding to the transmitted user ID from stored calculated data, i.e. data about body composition indicators (body fat percentages) and user IDs corresponding to the body composition indicators and transmits data about the selected calculated data, i. e. body composition indicator (body fat percentage) and the user ID corresponding to the body composition indicator to the PC 13 (STEP S13).

Then, when the PC 13 receives the transmitted data, it displays the body composition indicator (body fat percentage) of the target user (subject) in the display section 13a (STEP S14).

In the above-described body composition indicator estimation system in Example 1, the basic body indicator measurement device 11 measures basic body indicators by working with the mobile relay key 12, the correlation equation for calculating a body composition indicator can be changed on the administrator side 20, and data is processed based on a user ID from the mobile relay key 12. Accordingly, the body composition indicator estimation system in Example 1 can always provide the best body composition indicator to a user (subject) more conveniently without imposing a burden on the user.

### Example 2

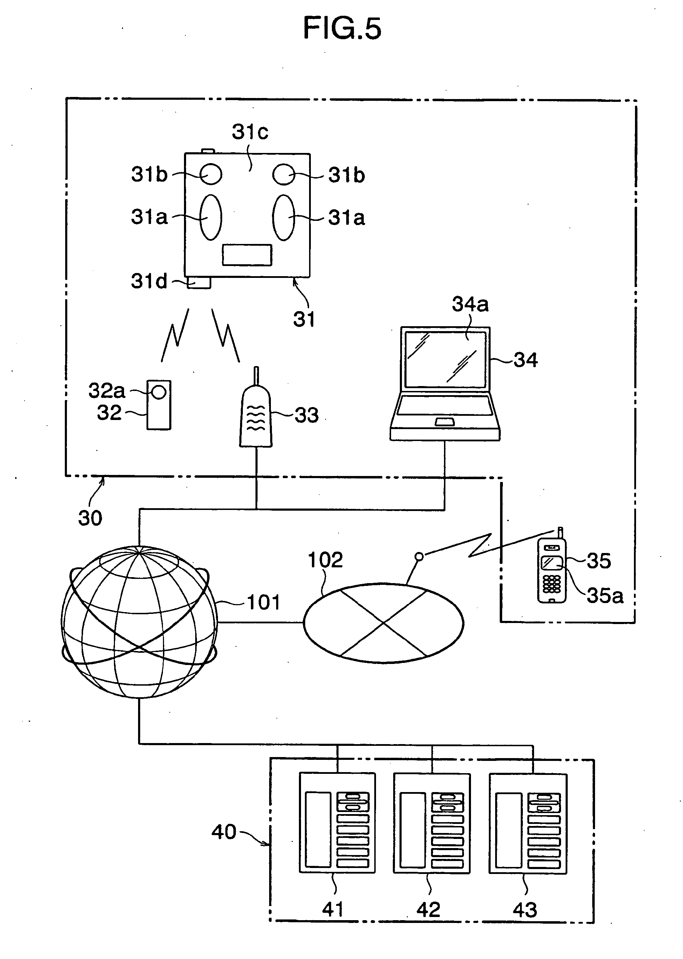

First, the configuration of a body composition indicator estimation system as Example 2 of the present invention will be specifically described by use of a system configuration diagram in Fig. 5 illustrating the configuration of the body composition indicator estimation system.

The body composition indicator estimation system in Example 2 comprises a basic body indicator measurement device 31, a mobile ID transmission key 32, a relay terminal 33, a PC (Personal Computer) 34 and/or a cellular phone 35 on a user side 30 and comprises a basic body indicator storage server 41, a body composition indicator computation server 42 and a body composition indicator storage server 43 on an administrator (provider) side 40. Further, the relay terminal 33, PC 34 and cellular phone 35 and the servers 41, 42 and 43 are connected to an Internet 101 and a cellular phone communication network 102.

The basic body indicator measurement device 31 corresponds to the aforementioned basic body indicator measuring means 2 and has current-passing electrodes 31a and measuring electrodes 31b on the platform 31c of the scale and measures a body impedance and a body weight as basic body indicators. Further, the basic body indicator measurement device 31 also has wireless communication means 31d using an infrared ray, low-power radio or the like and thereby receives data (user ID) from the mobile ID transmission key 32 and transmits data (user ID, basic body indicators (body impedance, body weight)) to the relay terminal 33.

The mobile ID transmission key 32 corresponds to a part of the aforementioned basic body indicator relay means 3 and stores a user ID for identifying a user in advance. Further, the mobile ID transmission key 32 is approximately the size of a portable lighter, has wireless communication means using an infrared ray, low-power radio or the like and thereby transmits data (user ID) to the basic body indicator measurement device 31.

The relay terminal 33 corresponds to a part of the aforementioned basic body indicator relay means 3 and has wireless communication means using an infrared ray, low-power radio or the like and thereby communicates data (user ID, basic body indicators (body impedance, body weight)) with the basic body indicator measurement device 31. Further, the relay terminal 33 has storage means for storing the communication-related data (user ID, basic body indicators (body impedance, body weight)). In addition, the relay terminal 33 transmits the stored data (user ID, basic body indicators (body impedance, body weight)) to the body composition indicator computation server 42 via the Internet 101.

The basic body indicator storage server 41 corresponds to a part of the aforementioned body composition indicator acquiring means 6 and stores other basic body indicators (body height, sex, age) which provide a correlation with a body composition indicator together with the basic body indicators (body impedance, body weight) measured by the basic body indicator measurement device 31 and a user ID for identifying a user corresponding to the basic body indicators (body height, sex, age) and communicates the data with the body composition indicator computation server 42. The basic body indicators (body height, sex, age) and user ID to be stored can be input by various techniques. For example, they can be input through the input section of the basic body indicator storage server 41 or input from the user side via the Internet 101 or cellular phone communication network 102.

The body composition indicator computation server 42 corresponds to a part of the aforementioned body composition indicator acquiring means 6 and stores a correlation equation of basic body indicators (body impedance, body weight, body height, sex, age) and body composition indicator (body fat percentage), calculates a body composition indicator (body fat percentage) by substituting the basic body indicators (body impedance, body weight) transmitted from the relay terminal 33 together with the user ID via the Internet 101 and the basic body indicators (body height, sex, age) transmitted from the basic body indicator storage server 41 together with the user ID into the correlation equation of basic body indicators (body impedance, body weight, body height, sex, age) and body composition indicator (body fat percentage), and transmits the calculated body composition indicator (body fat percentage) to the body composition indicator storage server 43 together with the user ID. Further, the body composition indicator computation server 42 corresponds to the aforementioned correlation equation changing means 5 and makes the stored correlation equation of basic body indicators (body impedance, body weight, body height, sex, age) and body composition indicator (body fat percentage) changeable on the administrator (provider) side 40. For example, it can change constants in the correlation equation of basic body indicators (body impedance, body weight, body height, sex, age) and body composition indicator (body fat percentage) or replace the correlation equation with a correlation equation of basic body indicators (body impedance, body weight, body height, sex, age) and body composition indicator (visceral fat area).

The body composition indicator storage server 43 stores the body composition indicator (body fat percentage) and user ID for identifying a user corresponding to the body composition indicator (body fat percentage) which have been transmitted from the body composition indicator computation server 42 and transmits the stored body composition indicator (body fat percentage) based on a request from the PC 34 or cellular phone 35.

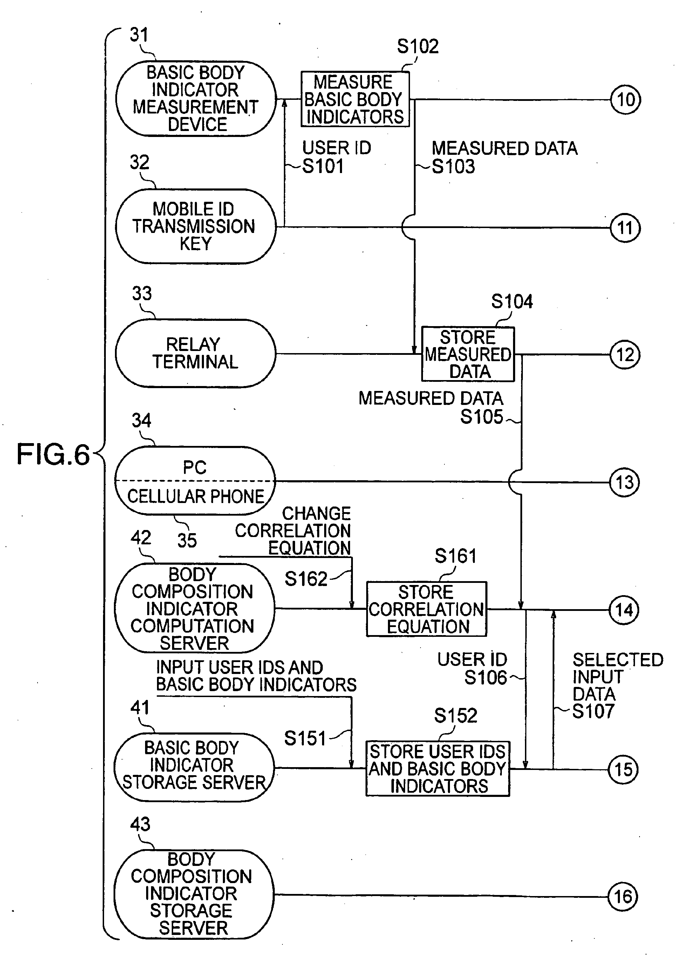

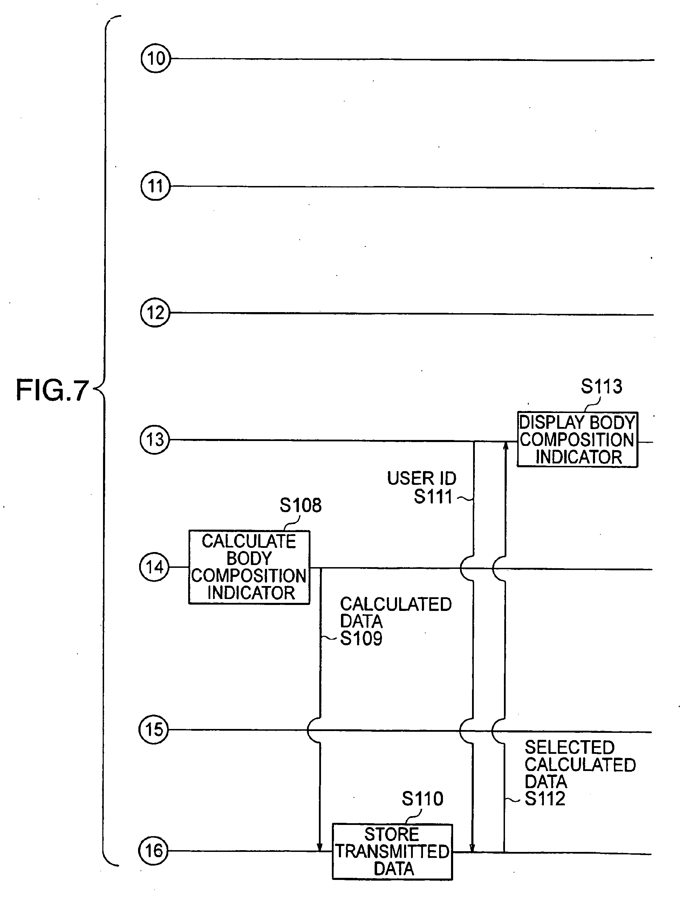

Next, a flow of data based on the operation of the body composition indicator estimation system according to the present invention will be specifically described by use of flowcharts in Figs. 6 and 7 illustrating a flow of data based on the operation of the body composition indicator estimation system.

Figs. 6 and 7 illustrate, from the top to the bottom, flows of processes in the basic body indicator measurement device 31, mobile ID transmission key 22, relay terminal 33, PC 34 or cellular phone 35, body composition indicator computation server 42, basic body indicator storage server 41 and body composition indicator storage server 43 and illustrate flows of data based on mutual communication by connecting these process routes by lines. Further, although an end terminal is not particularly illustrated, the following processes are repeated as appropriate.

First, when a user (subject) presses the communication button 32a of the mobile ID transmission key 32, the mobile ID transmission key 32 transmits data about a user ID and the like to the basic body indicator measurement device 31 wirelessly (STEP S101).

Then, the basic body indicator measurement device 31 is activated automatically based on the transmitted data, measures basic body indicators (body weight, body impedance) (STEP S102), and transmits data about the measured basic body indicators (body weight, body impedance) and a user ID corresponding to the basic body indicators (i.e. user ID transmitted from the mobile ID transmission key 32) to the relay terminal 33 wirelessly (STEP 5103) .

Then, the relay terminal 33 receives and stores the transmitted data (STEP S104) and transmits the stored data to the body composition indicator computation server 42 via the Internet 101 (STEP S105).

Then, when the body composition indicator computation server 42 receives the data from the relay terminal 33 via the Internet 101, it transmits data about the user ID to the basic body indicator storage server 41 (STEP S106).

Then, when the basic body indicator storage server 41 receives the transmitted data about the user ID, it selects basic body indicators (body height, sex, age) corresponding to the transmitted user ID from input data which has been input (STEP S151) and stored (STEP S152) in advance, i.e. data about basic body indicators (body height, sex, age) and user IDs corresponding to the basic body indicators and transmits the selected input data, i.e. data about the basic body indicators (body height, sex, age) and the user ID corresponding to the basic body indicators to the body composition indicator computation server 42 (STEP S107).

Then, when the body composition indicator computation server 42 receives the transmitted data, it calculates a body composition indicator (body fat percentage) by substituting the basic body indicators (body impedance, body weight) transmitted from the relay terminal 33 via the Internet 101 in STEP S105 and the basic body indicators (body height, sex, age) transmitted from the basic body indicator storage server 41 in STEP S107 into a correlation equation of basic body indicators (body impedance, body weight, body height, sex, age) and body composition indicator (body fat percentage) which has been stored (STEP S161) in advance (STEP S108) and transmits data about the calculated body composition indicator (body fat percentage) and the user ID corresponding to the body composition indicator to the body composition indicator storage server 43 (STEP S109). The correlation equation stored in STEP S161 is changed as appropriate by changing constants in the correlation equation of basic body indicators (body impedance, body weight, body height, sex, age) and body composition indicator (body fat percentage) or replacing the correlation equation with a correlation equation of basic body indicators (body impedance, body weight, body height, sex, age) and body composition indicator (visceral fat area) on the administrator (provider) side 40 (STEP S162) .

Then, the body composition indicator storage server 43 receives and stores the transmitted data (STEP S110).

Then, when the user ID of the target user (subject) is entered in the PC 34 or cellular phone 35 by the user (subject or a person who supervises the subject (such as a doctor or a family member living separately)) to view the measurement results, the PC 34 or cellular phone 35 transmits data about the entered user ID to the body composition indicator storage server 43 (STEP S111).

Then, when the body composition indicator storage server 43 receives the transmitted data, it selects a body composition indicator (body fat percentage) corresponding to the transmitted user ID from stored calculated data, i.e. data about body composition indicators (body fat percentages) and user IDs corresponding to the body composition indicators and transmits data about the selected calculated data, i.e. body composition indicator (body fat percentage) and the user ID corresponding to the body composition indicator to the PC 34 (STEP S112).

Then, when the PC 34 or cellular phone 35 receives the transmitted data, it displays the body composition indicator (body fat percentage) of the target user (subject) in the display section 34a or 35a (STEP S113).

In the above-described body composition indicator estimation system in Example 2, the basic body indicator measurement device 31 measures basic body indicators by working with the mobile ID transmission key 32, the correlation equation for calculating a body composition indicator can be changed on the administrator side, and data is processed based on a user ID from the mobile ID transmission key 32. Thus, the body composition indicator estimation system in Example 2 can always provide the best body composition indicator to a user (subject) more conveniently without imposing a burden on the user.

Although a body impedance, body weight, body height, sex and age have been presented as examples of the basic body indicators in the above Examples 1 and 2, the basic body indicators are not limited to these parameters. Further, although a body fat percentage and visceral fat area have been presented as examples of the body composition indicator, it is also possible to estimate a muscle amount, body water amount or bone mass. In addition, when a new indicator is available as the body composition indicator, it is also possible to estimate the new indicator.

## Claims

1. A body composition indicator estimation system comprising:
a body indicator acquiring unit (1) which acquires a body indicator,
a body indicator management unit (4) which manages the body indicator acquired by the body indicator acquiring unit (1), and
a body indicator confirmation terminal unit (7) which outputs the body indicator managed by the body indicator management unit (4) to a user, the body indicator confirmation terminal unit (7) comprising body composition indicator confirmation terminal means (8) for outputting the body composition indicator transmitted from the body composition indicator acquiring means (6) to the user;
these units being connected via a telecommunication network (100),
wherein
the body indicator acquiring unit (1) comprises:
basic body indicator measuring means (2) for measuring basic body indicators which provide a correlation with a body composition indicator, and
basic body indicator relay means (3) for transmitting the basic body indicators measured by the basic body indicator measuring means (2) to the body indicator management unit (4) via the telecommunication network (100),
**characterized in that** the body indicator management unit (4) comprises:
body composition indicator acquiring means (6) for calculating a body composition indicator, the body composition indicator being a body fat percentage, based on the basic body indicators transmitted from the basic body indicator relay means (3), the basic body indicators being body impedance, body weight, body height, sex, and
age, in accordance with a correlation equation of the basic body indicators and the body composition indicator which is stored in the body composition indicator acquiring means (6) and transmitting the calculated body composition indicator to the body indicator confirmation terminal unit (7) via the telecommunication network (100), and
correlation equation changing means (5) for changing the correlation equation stored in the body composition indicator acquiring means (6), wherein constants of the correlation equation of the basic body indicators and the body composition indicator are changeable, or for replacing the correlation equation with a correlation equation of the basic body indicators and the body composition indicator, wherein
the body composition indicator is a visceral fat area,

2. The system of claim 1, wherein
the basic body indicator relay means (3) comprises:
a mobile relay key (12) which stores the basic body indicators measured by the basic body indicator measuring means (2) and a user ID which corresponds to the basic body indicators, and
a relay terminal which transmits the basic body indicators and user ID stored in the mobile relay key (12) to the body composition indicator acquiring means (6) via the telecommunication network (100),
the basic body indicator measuring means (2) measures the basic body indicators by working with the mobile relay key (12), and
the mobile relay key (12) is portable and formed independently of the basic body indicator measuring means (2) and the relay terminal.

3. The system of claim 1 or 2, wherein
the basic body indicator measuring means (2) further comprises a mobile ID transmission key (32) which transmits a user ID,
the basic body indicator measuring means (2) measures the basic body indicators by working with the mobile ID transmission key (32),
the basic body indicator relay means (3) comprises a relay terminal which stores the basic body indicators measured by the basic body indicator measuring means (2) and a user ID which corresponds to the basic body indicators and transmits the stored basic body indicators and user ID to the body composition indicator acquiring means (6) via the telecommunication network (100), and
the mobile ID transmission key (32) is portable and formed independently of the basic body indicator measuring means (2) and the relay terminal.

4. The system of claim 1, 2 or 3, wherein
the body composition indicator confirmation terminal means (8) requests a body composition indicator corresponding to the user ID from the body composition indicator acquiring means (6) via the telecommunication network (100), and
the body composition indicator acquiring means (6) comprises:
a basic body indicator storage server (21) which stores other basic body indicators which provide a correlation with a body composition indicator together with the basic body indicators measured by the basic body indicator measuring means (2) and a user ID which corresponds to the basic body indicators,
a body composition indicator computation server (22) which calculates a body composition indicator based on the basic body indicators transmitted from the relay terminal together with the user ID via the telecommunication network (100) and the basic body indicators stored in the basic body indicator storage server (21) together with the user ID in accordance with the correlation equation, and
a body composition indicator storage server (23) which stores the body composition indicator calculated by the body composition indicator computation server (22) and
the user ID corresponding to the body composition indicator and transmits the stored body composition indicator based on a request from the body composition indicator confirmation terminal means (8).

## Patentansprüche

1. Schätzsystem für Körperzusammensetzungen, das umfasst:
eine Körperindikator-Erfassungseinheit (1), die einen Körperindikator erfasst,
eine Körperindikator-Verwaltungseinheit (4), die den Körperindikator verwaltet, der von der Körperindikator-Erfassungseinheit (1) erfasst wird, und
eine Körperindikator-Bestätigungsendgeräteinheit (7), die den von der Körperindikator-Verwaltungseinheit (4) verwalteten Körperindikator an einen Benutzer ausgibt, wobei die Körperindikator-Bestätigungsendgeräteinheit (7) ein Körperzusammensetzungsindikator-Bestätigungsendgerätemittel (8) zum Ausgeben des von dem Körperindikator-Erfassungsmittel (6) übermittelten Körperzusammensetzungsindikators an den Benutzer umfasst;
wobei diese Einheiten über ein Telekommunikationsnetz (100) verbunden sind,
wobei
die Körperindikator-Erfassungseinheit (1) umfasst:
Messmittel (2) für grundlegende Körperindikatoren zum Messen von grundlegenden Körperindikatoren, die eine Korrelation mit einem Körperzusammensetzungsindikator bereitstellen, und
Weiterleitungsmittel (3) für grundlegende Körperindikatoren zum Übermitteln der von dem Messmittel (6) für grundlegende Körperindikatoren gemessenen grundlegenden Körperindikatoren über das Telekommunikationsnetz (100) an die Körperindikator-Verwaltungseinheit (4),
**dadurch gekennzeichnet, dass** die Körperindikator-Verwaltungseinheit (4) umfasst:
ein Körperzusammensetzungsindikator-Erfassungsmittel (6) zum Berechnen eines Körperzusammensetzungsindikators, wobei der Körperzusammensetzungsindikator ein Körperfettanteil ist, basierend auf den grundlegenden Körperindikatoren, die von dem Weiterleitungsmittel (3) für grundlegende Körperindikatoren übermittelt werden, wobei die grundlegenden Körperindikatoren die Körperimpedanz, das Körpergewicht, die Körpergröße, das Geschlecht und das Alter sind, gemäß einer Korrelationsgleichung der grundlegenden Körperindikatoren und des Körperzusammensetzungsindikators, der in dem Körperzusammensetzungsindikator-Erfassungsmittel (6) gespeichert ist, und zum Übermitteln des berechneten Körperzusammensetzungsindikators über das Telekommunikationsnetz (100) an die Körperindikator-Bestätigungsendgeräteinheit (7), und ein Korrelationsgleichungsänderungsmittel (5) zum Ändern der in dem Körperzusammensetzungsindikator-Erfassungsmittel (6) gespeicherten Korrelationsgleichung, wobei die Konstanten der Korrelationsgleichung der grundlegenden Körperindikatoren und des Körperzusammensetzungsindikators änderbar sind, oder zum Ersetzen der Korrelationsgleichung durch eine Korrelationsgleichung der grundlegenden Körperindikatoren und des Körperzusammensetzungsindikators, wobei der Körperzusammensetzungsindikator ein viszeraler Fettbereich ist.

2. System nach Anspruch 1, wobei
das Weiterleitungsmittel (3) für grundlegende Körperindikatoren umfasst:
einen mobilen Weiterleitungsschlüssel (12), der die von dem Messmittel (2) für grundlegende Körperindikatoren gemessenen grundlegenden Körperindikatoren und eine Benutzer-ID bzw. -kennung, die den grundlegenden Körperindikatoren entspricht, speichert, und
ein Weiterleitungsendgerät, das die grundlegenden Körperindikatoren und die Benutzerkennung, die in dem mobilen Weiterleitungsschlüssel (12) gespeichert sind, über das Telekommunikationsnetz an das Körperzusammensetzungsindikator-Erfassungsmittel (6) übermittelt,
wobei das Messmittel (2) für grundlegende Körperindikatoren die grundlegenden Körperindikatoren durch Arbeiten mit dem mobilen Weiterleitungsschlüssel (12) misst, und
der mobile Weiterleitungsschlüssel (12) tragbar ist und unabhängig von dem Messmittel (2) für grundlegende Körperindikatoren und dem Weiterleitungsendgerät ausgebildet ist.

3. System nach Anspruch 1 oder 2, wobei
das Messmittel (2) für grundlegende Körperindikatoren ferner einen mobilen Kennungsübermittlungsschlüssel (32) umfasst, der eine Benutzerkennung übermittelt,
das Messmittel (2) für grundlegende Körperindikatoren die grundlegenden Körperindikatoren misst, indem es mit dem mobilen Kennungsübermittlungsschlüssel (32) arbeitet,
das grundlegende Körperindikator-Weiterleitungsmittel (3) ein Weiterleitungsendgerät umfasst, das die von dem Messmittel (2) für grundlegende Körperindikatoren gemessenen grundlegenden Körperindikatoren und eine Benutzerkennung, die den grundlegenden Körperindikatoren entspricht, speichert und die gespeicherten grundlegenden Körperindikatoren und die Benutzerkennung über das Telekommunikationsnetz (100) an das Körperzusammensetzungsindikator-Erfassungsmittel (6) übermittelt, und
der mobile Kennungsübermittlungsschlüssel (32) tragbar ist und unabhängig von dem Messmittel (2) für grundlegende Körperindikatoren und dem Weiterleitungsendgerät ausgebildet ist.

4. System nach Anspruch 1, 2 oder 3, wobei
das Körperzusammensetzungsindikator-Bestätigungsendgerätemittel (8) über das Telekommunikationsnetz (100) einen der Benutzerkennung entsprechenden Körperzusammensetzungsindikator von dem Körperzusammensetzungsindikator-Erfassungsmittel (6) anfordert und das Körperzusammensetzungsindikator-Erfassungsmittel (6) umfasst:
einen Speicherserver (21) für grundlegende Körperindikatoren, der andere grundlegende Körperindikatoren, welche eine Korrelation mit einem Körperzusammensetzungsindikator bereitstellen, zusammen mit den von dem Messmittel (2) für grundlegende Körperindikatoren gemessenen grundlegenden Körperindikatoren und einer den grundlegenden Körperindikatoren entsprechenden Benutzerkennung speichert,
einen Körperzusammensetzungsindikator-Berechnungsserver (22), der einen Körperzusammensetzungsindikator basierend auf den von dem Weiterleitungsendgerät zusammen mit der Benutzerkennung über das Telekommunikationsnetz (100) übermittelten grundlegenden Körperindikatoren und den in dem Speicherserver (21) für grundlegende Körperindikatoren zusammen mit der Benutzerkennung gespeicherten grundlegenden Körperindikatoren gemäß der Korrelationsgleichung berechnet, und
einen Körperzusammensetzungsindikator-Speicherserver (23), der den von dem Körperzusammensetzungsindikator-Berechnungsserver (22) berechneten Körperzusammensetzungsindikator und die dem Körperzusammensetzungsindikator entsprechenden Benutzerkennung speichert und den gespeicherten Körperzusammensetzungsindikator basierend auf einer Anforderung von dem Körperzusammensetzungsindikator-Bestätigungsendgerätemittel (8) übermittelt.

## Revendications

1. Système d'estimation d'indicateur de composition corporelle comprenant :
une unité d'acquisition d'indicateur corporel (1) qui acquiert un indicateur corporel,
une unité de gestion d'indicateur corporel (4) qui gère l'indicateur corporel acquis par l'unité d'acquisition d'indicateur corporel (1), et
une unité de terminal de confirmation d'indicateur corporel (7) qui délivre l'indicateur corporel géré par l'unité de gestion d'indicateur corporel (4) à un utilisateur, l'unité de terminal de confirmation d'indicateur corporel (7) comprenant des moyens de terminal de confirmation d'indicateur de composition corporelle (8) pour délivrer l'indicateur de composition corporelle transmis par les moyens d'acquisition d'indicateur de composition corporelle (6) à l'utilisateur;
ces unités étant connectées par l'intermédiaire d'un réseau de télécommunication (100),
dans lequel
l'unité d'acquisition d'indicateur corporel (1) comprend :
des moyens de mesure d'indicateur corporel de base (2) pour mesurer des indicateurs corporels de base qui fournissent une corrélation avec un indicateur de composition corporelle, et
des moyens de relais d'indicateur corporel de base (3) pour transmettre les indicateurs corporels de base mesurés par les moyens de mesure d'indicateur corporel de base (2) à l'unité de gestion d'indicateur corporel (4) par l'intermédiaire du réseau de télécommunication (100),
**caractérisé en ce que** l'unité de gestion d'indicateur corporel (4) comprend :
des moyens d'acquisition d'indicateur de composition corporelle (6) pour calculer un indicateur de composition corporelle, l'indicateur de composition corporelle étant un pourcentage de graisse corporelle, sur la base des indicateurs corporels de base transmis par les moyens de relais d'indicateur corporel de base (3), les indicateurs corporels de base étant l'impédance corporelle, le poids du corps, la hauteur du corps, le sexe et l'âge, conformément à une équation de corrélation des indicateurs corporels de base et de l'indicateur de composition corporelle qui est mémorisé dans les moyens d'acquisition d'indicateur de composition corporelle (6) et transmettre l'indicateur de composition corporelle calculé à l'unité de terminal de confirmation d'indicateur corporel (7) par l'intermédiaire du réseau de télécommunication (100), et
des moyens de modification d'équation de corrélation (5) pour modifier l'équation de corrélation mémorisée dans les moyens d'acquisition d'indicateur de composition corporelle (6), dans lequel les constantes de l'équation de corrélation des indicateurs corporels de base et de l'indicateur de composition corporelle peuvent être modifiées, ou pour remplacer l'équation de corrélation par une équation de corrélation des indicateurs corporels de base et de l'indicateur de composition corporelle, dans laquelle l'indicateur de composition corporelle est une surface de graisse viscérale.

2. Système selon la revendication 1, dans lequel
les moyens de relais d'indicateur corporel de base (3) comprennent :
une clé de relais mobile (12) qui mémorise les indicateurs corporels de base mesurés par les moyens de mesure d'indicateur corporel de base (2) et un identifiant d'utilisateur qui correspond aux indicateurs corporels de base, et
un terminal de relais qui transmet les indicateurs corporels de base et l'identifiant d'utilisateur mémorisés dans la clé de relais mobile (12) aux moyens d'acquisition d'indicateur de composition corporelle (6) par l'intermédiaire du réseau de télécommunication (100),
les moyens de mesure d'indicateur corporel de base (2) mesurent les indicateurs corporels de base en travaillant avec la clé de relais mobile (12), et
la clé de relais mobile (12) est portable et formée indépendamment des moyens de mesure d'indicateur corporel de base (2) et du terminal de relais.

3. Système selon la revendication 1 ou 2, dans lequel
les moyens de mesure d'indicateur corporel de base (2) comprennent en outre une clé de transmission d'identifiant mobile (32) qui transmet un identifiant d'utilisateur,
les moyens de mesure d'indicateur corporel de base (2) mesurent les indicateurs corporels de base en travaillant avec la clé de transmission d'identifiant mobile (32),
les moyens de relais d'indicateur corporel de base (3) comprennent un terminal de relais qui mémorise les indicateurs corporels de base mesurés par les moyens de mesure d'indicateur corporel de base (2) et un identifiant d'utilisateur qui correspond aux indicateurs corporels de base et transmet les indicateurs corporels de base et l'identifiant d'utilisateur mémorisés aux moyens d'acquisition d'indicateur de composition corporelle (6) par l'intermédiaire du réseau de télécommunication (100), et
la clé de transmission d'identifiant mobile (32) est portable et formée indépendamment des moyens de mesure d'indicateur corporel de base (2) et du terminal de relais.

4. Système selon la revendication 1, 2 ou 3, dans lequel
les moyens de terminal de confirmation d'indicateur de composition corporelle (8) demandent un indicateur de composition corporelle correspondant à l'identifiant d'utilisateur aux moyens d'acquisition d'indicateur de composition corporelle (6) par l'intermédiaire du réseau de télécommunication (100), et
les moyens d'acquisition d'indicateur de composition corporelle (6) comprennent :
un serveur de mémorisation d'indicateur corporel de base (21) qui mémorise d'autres indicateurs corporels de base qui fournissent une corrélation avec un indicateur de composition corporelle avec les indicateurs corporels de base mesurés par les moyens de mesure d'indicateur corporel de base (2) et un identifiant d'utilisateur qui correspond aux indicateurs corporels de base,
un serveur de calcul d'indicateur de composition corporelle (22) qui calcule un indicateur de composition corporelle sur la base des indicateurs corporels de base transmis par le terminal de relais avec l'identifiant d'utilisateur par l'intermédiaire du réseau de télécommunication (100) et des indicateurs corporels de base mémorisés dans le serveur de mémorisation d'indicateur corporel de base (21) avec l'identifiant d'utilisateur conformément à l'équation de corrélation, et
un serveur de mémorisation d'indicateur de composition corporelle (23) qui mémorise l'indicateur de composition corporelle calculé par le serveur de calcul d'indicateur de composition corporelle (22) et l'identifiant d'utilisateur correspondant à l'indicateur de composition corporelle et transmet l'indicateur de composition corporelle mémorisé sur la base d'une demande des moyens de terminal de confirmation d'indicateur de composition corporelle (8).
